(19) Europäisches Patentamt
European Patent Office
Office européen des brevets

(11) **EP 4 714 360 A1**

(12) **EUROPEAN PATENT APPLICATION**

(43) Date of publication:
25.03.2026 Bulletin 2026/13

(21) Application number: 25173257.4

(22) Date of filing: 29.04.2025

(51) International Patent Classification (IPC):
*A61B 6/50* (2024.01)   *A61B 6/00* (2024.01)

(52) Cooperative Patent Classification (CPC):
A61B 6/5217; A61B 6/504

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 20.09.2024 KR 20240127653

(71) Applicant: Medipixel, Inc.
Seoul 06174 (KR)

(72) Inventors:
• LEE, Yong-Hee
  06174 Seoul (KR)
• KANG, Min-Yeong
  06174 Seoul (KR)
• WON, Donghyun
  06174 Seoul (KR)

(74) Representative: Studio Torta S.p.A.
Via Viotti, 9
10121 Torino (IT)

(54) **METHOD AND ELECTRONIC DEVICE FOR CALCULATING RATIO OF BLOOD FLOW BY VESSEL USING VASCULAR IMAGE**

(57)    A method, performed by at least one processor, for calculating a ratio of blood-flow volumes by vessel using a vascular image, includes acquiring at least one vascular image, classifying, in the at least one vascular image, a plurality of vessels, extracting geometric information of each of the plurality of vessels from the at least one vascular image, and calculating a ratio of blood-flow volumes by vessel based on a value extracted as the geometric information of each of the plurality of vessels and a reference value for the geometric information of each of the plurality of vessels, wherein the reference value are set based on values calculated from a plurality of subjects.

| VASCULAR IMAGE 110 | → | ELECTRONIC DEVICE 100 | → | RATIO OF BLOOD-FLOW VOLUMES BY VESSEL 120 |

**FIG. 1**

EP 4 714 360 A1

## Description

## BACKGROUND

**Field**

The present disclosure relates to a method of calculating a ratio of blood-flow volumes by vessel using a vascular image and to an electronic device.

**Description of Related Art**

A coronary artery is an artery encircling the heart and may supply blood to myocardium, which is muscular tissue of the heart. The amount of myocardium connected to a coronary artery may correspond to an amount of blood flowing through the artery, that is, a blood-flow volume. Accordingly, a disease that occurs in a coronary artery practically can become problematic for a patient when the blood-flow volume supplied is insufficient relative to the myocardium mass.

One or more methods for measuring myocardium mass includes directly capturing cross-sections of an entire heart, tracking regions corresponding to myocardium in all images, and summing the tracked regions. However, this method requires computed-tomography imaging that is not essential for a procedure and requires additional processes such as vessel segmentation to trace a myocardial territory (or territory) or myocardium mass supplied by each vessel.

Blood-flow volume is used when fractional flow reserve (FFR) is calculated, yet some blood-flow-volume calculation methods do not reflect a percentage of the whole myocardium that each vessel supplies. For example, the blood-flow volume may be calculated by estimating a reference diameter of the target vessel, calculating a reference area based on the estimated diameter, and multiplying the calculated area by an assumed blood velocity. However, this calculation method cannot reflect a vessel type, a difference in allocated myocardium percentage relative to other vessels, a total vessel volume, or a heart size of a patient. Therefore, a technology capable of calculating a ratio of blood-flow volumes by vessel using an actual interventional vascular image such as coronary angiography, without CT imaging, is required.

## SUMMARY

The present disclosure provides a method and an electronic device that calculate a ratio of blood-flow volumes by vessel using a vascular image in order to solve the above-described problems.

The present disclosure may be implemented in various forms, including a method, a device (system), and/or a non-transitory computer-readable recording medium storing computer-readable instructions.

In some implementations, a method, performed by at least one processor, for calculating a ratio of blood-flow volumes by vessel using a vascular image, may include acquiring at least one vascular image, classifying, in the at least one vascular image, a plurality of vessels, extracting geometric information of each of the plurality of vessels from the at least one vascular image, and calculating a ratio of blood-flow volumes by vessel based on a value extracted as the geometric information of each of the plurality of vessels and a reference value for the geometric information of each of the plurality of vessels, wherein the reference value are set based on values calculated from a plurality of subjects.

In some implementations, the geometric information may include at least one of a diameter, a length, a volume, a placement position, or a placement direction.

In some implementations, classifying the plurality of vessels may include classifying, in the at least one vascular image, a right coronary artery, a left anterior descending coronary artery, and a left circumflex coronary artery, or classifying, in the at least one vascular image, the right coronary artery and a left main coronary artery.

In some implementations, calculating the ratio of blood-flow volumes by vessel may include calculating a difference between the reference value and the extracted value for the geometric information of each of the plurality of vessels, and calculating, based on the calculated difference and reference distribution information of blood-flow volumes by vessel, the ratio of blood-flow volumes by vessel for each of the plurality of vessels.

In some implementations, the difference may include a ratio of the reference value to the extracted value or a ratio of a difference between the reference value and the extracted value to the extracted value.

In some implementations, the method may further include reconstructing, based on the at least one vascular image, a three-dimensional shape of each of the plurality of vessels.

In some implementations, the method may further include estimating, based on the ratio of blood-flow volumes by vessel, a blood-flow volume by vessel.

In some implementations, the method may further include providing a visual object corresponding to the calculated ratio of blood-flow volumes by vessel.

In some implementations, the at least one vascular image may include an image captured by coronary angiography.

In some implementations, an electronic device may include a memory, and at least one processor connected to the memory and configured to execute computer-readable instructions stored in the memory, wherein the at least one processor is configured to acquire at least one vascular image, classify, in the at least one vascular image, a plurality of vessels, extract geometric information of each of the plurality of vessels from the at least one vascular image, and calculate a ratio of blood-flow volumes by vessel based on a value extracted as the geometric information of each of the plurality of vessels and a reference value for the geometric information of each of the plurality of vessels, wherein the reference value are set based on values calculated from a plurality of subjects.

In some implementations, the at least one processor may be configured to calculate a difference between the reference value and the extracted value for the geometric information of each of the plurality of vessels, and calculate, based on the calculated difference and reference distribution information of blood-flow volumes by vessel, the ratio of blood-flow volumes by vessel for each of the plurality of vessels.

In some implementations, the at least one processor may be further configured to reconstruct a three-dimensional shape of the plurality of vessels based on the at least one vascular image.

In some implementations, the at least one processor may be further configured to estimate, based on the ratio of blood-flow volumes by vessel, a blood-flow volume by vessel.

In some implementations, a non-transitory computer-readable recording medium storing computer-readable instructions which, when executed by at least one processor, may cause the at least one processor to acquire at least one vascular image, classify, in the at least one vascular image, a plurality of vessels, extract geometric information of each of the plurality of vessels from the at least one vascular image, and calculate a ratio of blood-flow volumes by vessel based on a value extracted as the geometric information of each of the plurality of vessels and a reference value for the geometric information of each of the plurality of vessels, wherein the reference value are set based on values calculated from a plurality of subjects.

In some implementations, the instructions further cause the at least one processor to calculate a difference between the reference value and the extracted value for the geometric information of each of the plurality of vessels, and calculate, based on the calculated difference and reference distribution information of blood-flow volumes by vessel, the ratio of blood-flow volumes by vessel for each of the plurality of vessels.

In some implementations, the instructions may further cause the at least one processor to reconstruct a three-dimensional shape of the plurality of vessels based on the at least one vascular image.

In some implementations, the instructions may further cause the at least one processor to estimate, based on the ratio of blood-flow volumes by vessel, a blood-flow volume by vessel.

According to some aspects of the present disclosure, by using a vascular image employed in an actual interventional procedure without CT imaging, not only can cost be reduced but a ratio of blood-flow volumes by vessel can also be calculated more simply without unnecessary processes.

According to some aspects of the present disclosure, by using one or more reference values set based on values calculated from a plurality of subjects with respect to the geometric information of each of the plurality of vessels, the ratio of blood-flow volumes by vessel can be calculated without a process of calculating myocardium mass.

According to some aspects of the present disclosure, by using a blood-flow volume by vessel, a more accurate fractional flow reserve can be obtained, and consequently a more accurate lesion diagnosis can be supported.

Effects of the present disclosure are not limited to the effects mentioned above, and other effects not mentioned will be clearly understood by those of ordinary skill in the art from the description of the claims.

## BRIEF DESCRIPTION OF THE DRAWINGS

The embodiment(s) of the present disclosure will be described below with reference to the accompanying drawings, in which like reference numerals indicate like elements, but the embodiment(s) are not limited thereto.

FIG. 1 illustrates an electronic device for calculating a ratio of blood-flow volumes by vessel using a vascular image.
FIG. 2 is a diagram illustrating a configuration of the electronic device.
FIG. 3 is a diagram illustrating a configuration of a processor of the electronic device.
FIG. 4 is a diagram illustrating a method of extracting geometric information of each of a plurality of vessels classified in a vascular image.
FIG. 5 is a diagram illustrating a method of calculating a ratio of blood-flow volumes by vessel using a vascular image.
FIG. 6 is a diagram illustrating another method of calculating a ratio of blood-flow volumes by vessel using a vascular image.
FIG. 7 is a diagram illustrating a method of calculating a ratio of blood-flow volumes by vessel using geometric information of each of a plurality of vessels.
FIG. 8 is a diagram illustrating a method of calculating a ratio of blood-flow volumes by vessel using a diameter of each of a plurality of vessels.

FIG. 9 illustrates an artificial-neural-network model.

## DETAILED DESCRIPTION

Hereinafter, example details for the practice of the present disclosure will be described in detail with reference to the accompanying drawings. However, in the following description, detailed descriptions of well-known functions or configurations will be omitted if it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of components are omitted, it is not intended that such components are not included in any example.

Advantages and features of the disclosed examples and methods of accomplishing the same will be apparent by referring to examples described below in connection with the accompanying drawings. However, the present disclosure is not limited to the examples disclosed below, and may be implemented in various forms different from each other, and the examples are merely provided to make the present disclosure complete, and to fully disclose the scope of the disclosure to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Accordingly, the terms used in this disclosure should be defined based on the meaning of the term and the overall content of the present disclosure, rather than simply the name of the term.

As used herein, the singular forms "a," "an," and "the" are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it is intended as meaning that the portion may additionally comprise (or include or have) another component, rather than excluding the same, unless specified to the contrary.

Further, the term "module" or "unit" used herein refers to a software or hardware component, and "module" or "unit" performs certain roles. However, the meaning of the "module" or "unit" is not limited to software or hardware. The "module" or "unit" may be configured to be in an addressable storage medium or configured to play one or more processors. Accordingly, as an example, the "module" or "unit" may include components such as software components, object-oriented software components, class components, and task components, and at least one of processes, functions, attributes, procedures, subroutines, program code segments, drivers, firmware, micro-codes, circuits, data, database, data structures, tables, arrays, and variables. Furthermore, functions provided in the components and the "modules" or "units" may be combined into a smaller number of components and "modules" or "units", or further divided into additional components and "modules" or "units."

A "module" or "unit" may be implemented as a processor and a memory, or may be implemented as a circuit (circuitry). Terms such as circuit and circuitry may refer to circuits in hardware, but may also refer to circuits in software. The "processor" should be interpreted broadly to encompass a general-purpose processor, a central processing unit (CPU), a microprocessor, a digital signal processor (DSP), a neural processing unit (NPU), a controller, a microcontroller, a state machine, etc. Under some circumstances, the "processor" may refer to an application-specific integrated circuit (ASIC), a programmable logic device (PLD), a field-programmable gate array (FPGA), etc. The "processor" may refer to a combination for processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, etc. The memory is said to be in electronic communication with a processor if the processor can read information from and/or write information to the memory. The memory integrated with the processor is in electronic communication with the processor.

In addition, terms such as first, second, A, B, (a), (b), etc. used in the following examples are only used to distinguish certain components from other components, and the nature, sequence, order, etc. of the components are not limited by the terms.

In addition, in the following examples, if a certain component is stated as being "connected," "combined" or "coupled" to another component, it is to be understood that there may be yet another intervening component "connected," "combined" or "coupled" between the two components, although the two components may also be directly connected or coupled to each other.

In addition, as used in the following examples, "comprise" and/or "comprising" does not foreclose the presence or addition of one or more other elements, steps, operations, and/or devices in addition to the recited elements, steps, operations, or devices.

Hereinafter, various examples of the present disclosure will be described in detail with reference to the accompanying drawings.

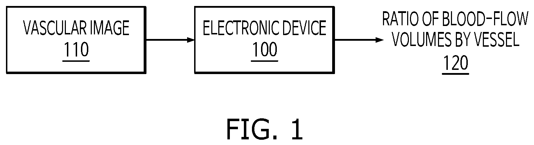

FIG. 1 is a diagram exemplarily illustrating an electronic device for calculating the ratio of blood flow volumes by vessel using a vascular image. Referring to FIG. 1, the electronic device 100 may calculate the ratio of blood flow volumes by vessel 120 using a vascular image 110. Here, the vascular image 110 may refer to an image (e.g., a video or still image) captured for the purpose of diagnosing, treating, or preventing disease, and specifically, may refer to an image of a patient's vasculature. According to an example, the vascular image 110 may include an image of a patient's vasculature captured in a state where a contrast agent has been administered to the patient. For example, the vascular image 110 may include an image acquired through coronary angiography. In addition, the vasculature included in the vascular image 110 may include coronary arteries. The coronary arteries are positioned in a manner that encircles the heart in a crown-like (coronary) fashion and may be subdivided based on their positional arrangement. For example, the coronary arteries may be categorized into a right coronary artery (RCA), which originates from the right side of the proximal portion of the ascending aorta and mainly runs along the right side of the heart, and a left coronary artery (LCA), which originates from the left side of the proximal portion of the ascending aorta and primarily runs along the left side of the heart. Furthermore, the left coronary artery may be further divided into a left main coronary artery (LMCA) (hereinafter referred to as LM), which begins at the upper left side of the heart, and branches into a left anterior descending coronary artery (LAD) and a left circumflex coronary artery (LCX). In the present disclosure, since the ratio of blood flow volumes by vessel can be calculated without myocardial information, the vascular image 110 need not be a three-dimensional image. For example, the vascular image 110 may include not only a three-dimensional image, but also a two-dimensional image, a projection image, or a contrast-enhanced image, among others.

Although a storage system communicable with the electronic device 100 is not illustrated in FIG. 1, the electronic device 100 may be configured to be connected to, or communicable with, at least one storage system. The storage system configured to be connected to, or communicable with, the electronic device 100 may include a device or a cloud system that stores and manages various data associated with calculating the ratio 120 of blood-flow volumes by vessel using the vascular image 110. For efficient data management, the storage system may store and manage the various data using a database. The various data may include arbitrary data associated with calculating the ratio 120 of blood-flow volumes by vessel using the vascular image 110. For example, the various data may include a machine-learning model, training data, or the vascular image 110 associated with calculating the ratio 120 of blood-flow volumes by vessel using the vascular image 110, but the data are not limited thereto.

In calculating the ratio 120 of blood-flow volumes by vessel using the vascular image 110, the electronic device 100 may first acquire at least one vascular image 110. The vascular image 110 may be received through a communicable storage medium, for example, a hospital system or a local or cloud storage system.

Next, the electronic device 100 may classify a plurality of vessels in the at least one vascular image 110. For example, the electronic device 100 may classify the plurality of vessels in the at least one vascular image 110 by using a machine-learning model, for example, a vessel-classification model. In an example, the electronic device 100 may classify a right coronary artery, a left anterior descending coronary artery, and a left circumflex coronary artery in the at least one vascular image 110. In another example, the electronic device 100 may classify a right coronary artery and a left main coronary artery in the at least one vascular image 110.

Thereafter, the electronic device 100 may extract geometric information of each of the plurality of vessels from the at least one vascular image 110. The geometric information may include at least one of a diameter, a length, a volume, a placement position, or a placement direction.

Subsequently, the electronic device 100 may calculate the ratio 120 of blood-flow volumes by vessel based on a value extracted as the geometric information of each of the plurality of vessels. For example, the electronic device 100 may calculate a difference between a reference value for the geometric information of each of the plurality of vessels and a value extracted from the at least one vascular image 110. The reference value for the geometric information of each of the plurality of vessels may be set to an average of values calculated from a plurality of subjects. According to an example, the electronic device 100 may calculate the difference as a ratio of the reference value to the value extracted from the at least one vascular image 110 for the geometric information of each of the plurality of vessels. According to another example, the electronic device 100 may calculate the difference as a ratio of a difference between the reference value for the geometric information of each of the plurality of vessels and the value extracted from the at least one vascular image 110 to the reference value. The electronic device 100 may then calculate the ratio 120 of blood-flow volumes by vessel for each of the plurality of vessels based on the calculated difference and reference distribution information of blood-flow volumes by vessel. The reference distribution information of blood-flow volumes by vessel may be set to an average of values calculated from the plurality of subjects.

According to an example, the electronic device 100 may reconstruct a three-dimensional shape of the plurality of vessels based on the at least one vascular image 110. The electronic device 100 may then extract geometric information from each three-dimensional shape of the plurality of vessels. In this case, the geometric information may further include an area. For example, the geometric information may include at least one of a diameter, a length, a volume, an area, a placement position, or a placement direction.

After calculating the ratio 120 of blood-flow volumes by vessel, the electronic device 100 may estimate a blood-flow volume by vessel based on the ratio 120 of blood-flow volumes by vessel. For example, the electronic device 100 may set a weight according to the ratio 120 of blood-flow volumes by vessel and may apply the weight to the blood-flow volume by vessel.

According to an example, the electronic device 100 may provide a visual object corresponding to the ratio 120 of blood-flow volumes by vessel. The visual object may include at least one of text, a symbol, an image, or an animation indicating the ratio 120 of blood-flow volumes by vessel. For example, the electronic device 100 may display the visual object corresponding to the ratio 120 of blood-flow volumes by vessel so as to overlap the vascular image 110. **In** this case, the visual object corresponding to the ratio 120 of blood-flow volumes by vessel may be displayed so as to overlap an area in which the corresponding vessel is placed or an adjacent area among the vessels included in the vascular image 110. For example, when the ratio 120 of blood-flow volumes by vessel represents a ratio of blood-flow volumes of a right coronary artery, a left anterior descending coronary artery, and a left circumflex coronary artery and corresponds respectively to a first visual object, a second visual object, and a third visual object, the electronic device 100 may display the first visual object so as to overlap an area in which the right coronary artery is placed or an adjacent area, may display the second visual object so as to overlap an area in which the left anterior descending coronary artery is placed or an adjacent area, and may display the third visual object so as to overlap an area in which the left circumflex coronary artery is placed or an adjacent area. Alternatively, when the ratio 120 of blood-flow volumes by vessel represents a ratio of blood-flow volumes of a right coronary artery and a left main coronary artery and corresponds respectively to a first visual object and a second visual object, the electronic device 100 may display the first visual object so as to overlap an area in which the right coronary artery is placed or an adjacent area and may display the second visual object so as to overlap an area in which the left main coronary artery is placed or an adjacent area. In some implementations, when a vessel involved in calculation of the ratio 120 of blood-flow volumes by vessel is not included in the vascular image 110, the electronic device 100 may display the visual object corresponding to the ratio 120 of blood-flow volumes by vessel for not only vessels included in the vascular image 110 but also vessels not included.

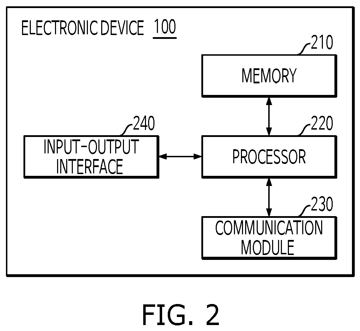

FIG. 2 is a diagram illustrating a configuration of the electronic device 100. Referring to FIG. 2, the electronic device 100 may include a memory 210, a processor 220, a communication module 230, and an input-output interface 240. However, the configuration of the electronic device 100 is not limited thereto. According to various implementations, the electronic device 100 may omit at least one of the above-described components and may further include at least one other component. In an example, the electronic device 100 may further include a display. In this case, the electronic device 100 may display a vascular image captured from a vessel, for example, the vascular image 110 of FIG. 1, on the display.

The memory 210 may store various data used by at least one other component of the electronic device 100, for example, the processor 220. The data may include, for example, software or a program and input or output data related to instructions.

The memory 210 may include any non-transitory computer-readable recording medium. According to an example, the memory 210 may include a non-volatile mass-storage device such as a disk drive, a solid-state drive, or flash memory. In another example, a non-volatile mass-storage device such as read-only memory, a solid-state drive, flash memory, or a disk drive may be included in the electronic device 100 as a separate non-volatile storage device distinguished from the memory 210. The memory 210 may store an operating system and at least one program code, for example, instructions such as calculating the ratio of blood-flow volumes by vessel. Although the memory 210 is illustrated as a single memory in FIG. 2 for convenience of description, the memory 210 may include a plurality of memories or buffer memories.

Software components may be loaded from a computer-readable recording medium separate from the memory 210. Such a separate computer-readable recording medium may include a recording medium directly connectable to the electronic device 100, for example, a floppy drive, a disk, a tape, a DVD/CD-ROM drive, or a memory card. In another example, the software components may be loaded to the memory 210 through the communication module 230 rather than the computer-readable recording medium. For example, at least one program may be loaded to the memory 210 based on a computer program installed by files provided through the communication module 230 by developers or by a file-distribution system that distributes installation files of an application, for example, a program for transmitting data such as a vascular image in which a vessel is captured.

The processor 220 may control at least one other component of the electronic device 100, for example, a hardware or software component connected to the processor 220, by executing software and may perform various data processing or calculations. According to an example, as at least part of the data processing or calculations, the processor 220 may load instructions or data received from another component, for example, the communication module 230, into a volatile memory, may process the instructions or data stored in the volatile memory, and may store result data in a non-volatile memory.

The processor 220 may be configured to process computer-program instructions by performing basic arithmetic, logic, and input-output operations. The instructions may be provided to the electronic device 100 or another external system by the memory 210 or by the communication module 230. For example, the processor 220 may calculate the ratio of blood-flow volumes by vessel using a vascular image. The processor 220 may then store the calculated ratio of blood-flow volumes by vessel in the memory 210, display the ratio on a display of the electronic device 100, or transmit the ratio to an external electronic device through the communication module 230. Alternatively, the processor 220 may perform an additional analysis operation such as estimating a blood-flow volume by vessel by using the ratio of blood-flow volumes by vessel. Although the processor 220 is illustrated as a single processor in FIG. 2 for convenience of description, the processor 220 may include a plurality of processors.

The communication module 230 may support establishment of a direct, for example, wired, communication channel or a wireless communication channel between the electronic device 100 and an external electronic device and may support communication through the established communication channel. For example, the communication module 230 may provide a configuration or a function for allowing the electronic device 100 and an external electronic device, for example, a user terminal or a cloud server, to communicate with each other through a network. In an example, a control signal, an instruction, or data that is provided by the processor 220 of the electronic device 100 may be transmitted to an external electronic device through the communication module 230 and a network via the communication module of the external electronic device. For example, the electronic device 100 may receive a vascular image in which a vessel of a subject is captured from an external electronic device through the communication module 230.

The input-output interface 240 may be means for interfacing between the electronic device 100 and an input or output device that is connected to, or may be included in, the electronic device 100 (not illustrated). For example, the input-output interface 240 may include at least one of a PCI Express interface or an Ethernet interface. Although the input-output interface 240 is illustrated as a component configured separately from the processor 220 in FIG. 2, the input-output interface 240 may be configured to be included in the processor 220.

According to an example, the processor 220 may perform a function related to calculating the ratio of blood-flow volumes by vessel using a vascular image. To perform the function related to calculating the ratio of blood-flow volumes by vessel, the processor 220 may execute at least one computer-readable program included in the memory 210. The at least one program may include instructions for acquiring at least one vascular image, classifying a plurality of vessels in the at least one vascular image, extracting geometric information of each of the plurality of vessels from the at least one vascular image, and calculating a ratio of blood-flow volumes by vessel based on a value extracted as the geometric information of each of the plurality of vessels. For convenience of description below, execution of the at least one program by the processor 220 to perform the function related to calculating the ratio of blood-flow volumes by vessel using a vascular image may be described as the processor 220 performing the function related to calculating the ratio of blood-flow volumes by vessel using a vascular image. For example, inclusion, in the at least one program, of instructions related to calculating the ratio of blood-flow volumes by vessel using a vascular image may correspond to a description that the processor 220 performs the function related to calculating the ratio of blood-flow volumes by vessel using a vascular image.

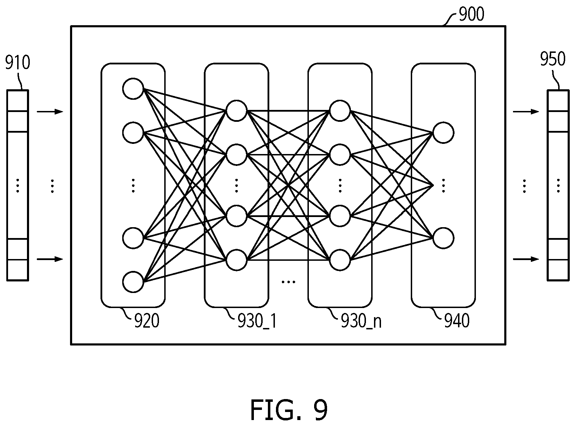

According to an example, the processor 220 may, through a machine-learning model that receives a vascular image as an input, obtain a ratio of blood-flow volumes by vessel. The machine-learning model may include any model used to infer an answer for a given input. According to an example, the machine-learning model may include an artificial-neural-network model including an input layer, a plurality of hidden layers, and an output layer. Each layer may include at least one node. The machine-learning model may include weights associated with the plurality of nodes included in the machine-learning model. The weights may include any parameter associated with the machine-learning model. The machine-learning model of the present disclosure may be a model trained by using various training methods. For example, various training methods such as supervised learning, semi-supervised learning, unsupervised learning, or reinforcement learning may be used in the present disclosure. In the present disclosure, the machine-learning model may refer to an artificial-neural-network model, and the artificial-neural-network model may refer to the machine-learning model. The artificial-neural-network model will be described in detail below with reference to FIG. 9.

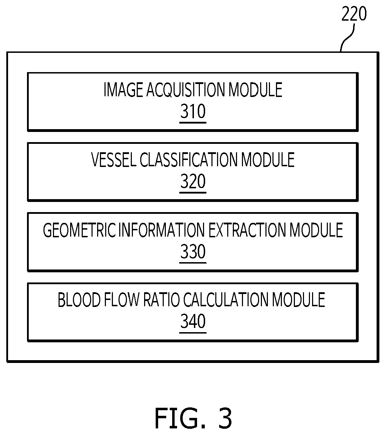

FIG. 3 is a diagram illustrating a configuration of the processor 220. Referring to FIG. 3, the processor 220 may calculate the ratio 120 of blood-flow volumes by vessel using a vascular image, for example, the vascular image 110 of FIG. 1. To this end, the processor 220 may include an image acquisition module 310, a vessel classification module 320, a geometric information extraction module 330, and a blood flow ratio calculation module 340. However, kinds of components included in the processor 220 are distinguished according to functions related to calculating the ratio of blood-flow volumes by vessel using a vascular image, and the kinds and the number of the components are not limited thereto. At least one of the components included in the processor 220 may be implemented in the form of instructions stored in the memory 210.

The image acquisition module 310 may acquire at least one vascular image in which a vessel is captured.

According to an example, the image acquisition module 310 may acquire at least one vascular image captured from a target patient while a contrast agent is administered to the target patient. For example, the vascular image may include an image captured by coronary angiography. Such a vascular image may be received from a storage system that is connected to, or communicable with, the electronic device 100, for example, a hospital system, an electronic-medical-record system, a prescription-delivery system, a medical-image system, a laboratory-information system, a local or cloud storage system, an internal memory, or a user terminal.

The vessel classification module 320 may classify a plurality of vessels in at least one vascular image acquired through the image acquisition module 310. For example, the vessel classification module 320 may classify the plurality of vessels in at least one vascular image by using a machine-learning model, for example, a vessel-classification model. In an example, the vessel classification module 320 may classify three major coronary arteries, that is, a right coronary artery, a left anterior descending coronary artery, and a left circumflex coronary artery, in at least one vascular image. In another example, the vessel classification module 320 may classify a right coronary artery and a left main coronary artery by broadly classifying coronary arteries in at least one vascular image.

The geometric information extraction module 330 may extract geometric information of each of the plurality of vessels from at least one vascular image. The geometric information may include at least one of a diameter, a length, a volume, a placement position, or a placement direction. According to an example, the geometric information extraction module 330 may measure a diameter of a proximal portion of each of the plurality of vessels in at least one vascular image.

The blood flow ratio calculation module 340 may calculate the ratio of blood-flow volumes by vessel based on a value extracted as the geometric information of each of the plurality of vessels. For example, the blood flow ratio calculation module 340 may calculate a difference between a reference value for the geometric information of each of the plurality of vessels and a value extracted from at least one vascular image. The reference value for the geometric information of each of the plurality of vessels may be set to an average of values calculated from a plurality of subjects. The blood flow ratio calculation module 340 may calculate the ratio of blood-flow volumes by vessel for each of the plurality of vessels based on the calculated difference and reference distribution information of blood-flow volumes by vessel. The reference distribution information of blood-flow volumes by vessel may be set to an average of values calculated from the plurality of subjects.

According to an example, the blood flow ratio calculation module 340 may calculate the difference between the reference value and the extracted value for the geometric information of each of the plurality of vessels as a ratio of the reference value to the extracted value, as in Equation 1.

$$\text{Equation 1}$$

$$D(x, x') = D_x = \frac{x}{x'}$$

Here, D represents a difference between the reference value for the geometric information of a vessel and the extracted value, x represents the reference value for the geometric information of the vessel, and x' may represent the value extracted as the geometric information of the vessel.

According to another example, the blood flow ratio calculation module 340 may calculate the difference between the reference value and the extracted value as a ratio of a difference between the reference value for the geometric information of each of the plurality of vessels and the value extracted from at least one vascular image to the value extracted from the at least one vascular image, as in Equation 2.

$$\text{Equation 2}$$

$$D(x, x') = D_x = \frac{x - x'}{x'}$$

Likewise, $D_x$ represents a difference between the reference value for the geometric information of vessel x and the extracted value, x represents the reference value for the geometric information of the vessel, and x' may represent the value extracted as the geometric information of the vessel.

However, the method of calculating the difference between the reference value and the extracted value for the geometric information of a vessel is not limited to Equations 1 and 2 above, and the difference may be calculated by using various other equations.

The blood flow ratio calculation module 340 may calculate a total required blood-flow volume of a target patient based on the difference between the reference value and the extracted value for the geometric information of a vessel by using Equation 3 below.

Equation 3

$$M_{myo} = F(D_A) + F(D_B) + F(D_C)$$

Here, $M_{myo}$ represents a total required blood-flow volume, $D_A$ represents a difference between a reference value and an extracted value for geometric information of vessel A, $D_B$ represents a difference between a reference value and an extracted value for geometric information of vessel B, $D_C$ represents a difference between a reference value and an extracted value for geometric information of vessel C, and the function F may represent a function that converts the difference between the reference value and the extracted value for geometric information of a vessel into a proportion of a blood-flow volume carried by the vessel.

**In** Equation 3, three vessels A, B, and C are summed when the total required blood-flow volume is calculated, but the calculation is not limited thereto. For example, A, B, and C may respectively represent a right coronary artery, a left anterior descending coronary artery, and a left circumflex coronary artery. Alternatively, when a right coronary artery and a left main coronary artery are used to calculate the total required blood-flow volume, A and B may respectively correspond to the right coronary artery and the left main coronary artery, and vessel C may be omitted from Equation 3.

The blood flow ratio calculation module 340 may calculate a blood-flow-volume ratio of a vessel by using Equation 4 below.

Equation 4

$$R_v = \frac{F(D_v)}{M_{myo}}$$

Here, $R_v$ represents a blood-flow-volume ratio of vessel v, $D_v$ represents a difference between a reference value and an extracted value for geometric information of vessel v, the function F represents a function that converts the difference between the reference value and the extracted value for geometric information of a vessel into a proportion of a blood-flow volume of the vessel, and $M_{myo}$ may represent the total required blood-flow volume.

In an example, the processor 220 may reconstruct a three-dimensional shape of a plurality of vessels based on at least one vascular image. The processor 220 may then extract geometric information from each three-dimensional shape of the plurality of vessels. In this case, the geometric information may further include an area. For example, the geometric information may include at least one of a diameter, a length, a volume, an area, a placement position, or a placement direction.

In an example, the processor 220 may calculate a ratio of blood-flow volumes by vessel of coronary arteries by using a diameter of a proximal portion of each coronary artery. The processor 220 may also calculate a ratio of blood-flow volumes by vessel of the coronary arteries by using a length of a partial section of each coronary artery. In addition, the processor 220 may calculate a volume by using a length of a partial section of each coronary artery and a diameter of a corresponding partial section and may calculate a ratio of blood-flow volumes by vessel of the coronary arteries by using the calculated volume. The processor 220 may also calculate a ratio of blood-flow volumes by vessel of the coronary arteries by using an area of a partial section of each coronary artery. Further, the processor 220 may sum entire lengths of the coronary arteries and may calculate a ratio of blood-flow volumes by vessel of the coronary arteries by using the summed value. The processor 220 may also sum entire volumes of the coronary arteries and may calculate a ratio of blood-flow volumes by vessel of the coronary arteries by using the summed value. The processor 220 may further sum entire areas of the coronary arteries and may calculate a ratio of blood-flow volumes by vessel of the coronary arteries by using the summed value.

In an example, the processor 220 may estimate a blood-flow volume by vessel based on the ratio of blood-flow volumes by vessel. For example, the processor 220 may set a weight according to the ratio of blood-flow volumes by vessel and may apply the weight to the blood-flow volume by vessel. A method of applying the weight set according to the ratio of blood-flow volumes by vessel to the blood-flow volume by vessel may be performed by using Equation 5 below.

Equation 5

$$Q' = w(R_v) * Q$$

Here, Q' denotes a blood-flow volume to which a weight is applied, Q denotes a blood-flow volume before the weight is applied, the function w is a function that sets the weight according to the ratio of blood-flow volumes by vessel, and

$R_v$ may represent a ratio of the blood-flow volume of vessel v.

According to an example, the processor 220 may provide a visual object corresponding to the ratio of blood-flow volumes by vessel. For example, the electronic device 100 may further include a display for presenting the visual object, and the processor 220 may provide, through the display, the visual object corresponding to the ratio of blood-flow volumes by vessel to a user. The visual object may include at least one of text, a symbol, an image, or an animation that represents the ratio of blood-flow volumes by vessel. For example, the processor 220 may display the visual object corresponding to the ratio of blood-flow volumes by vessel so as to overlap a vascular image. In this case, the visual object corresponding to the ratio of blood-flow volumes by vessel may be displayed so as to overlap an area in which the corresponding vessel is placed or an adjacent area among the vessels included in the vascular image. In some implementations, when a vessel involved in calculation of the ratio of blood-flow volumes by vessel is not included in the vascular image, the processor 220 may display the visual object corresponding to the ratio of blood-flow volumes by vessel for not only vessels included in the vascular image but also vessels not included.

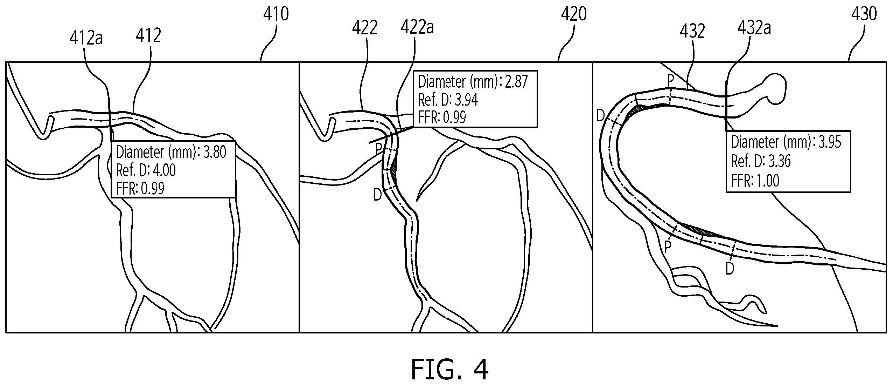

FIG. 4 is a diagram illustrating a method of extracting geometric information of each of a plurality of vessels classified in a vascular image. Referring to FIG. 4, a processor of an electronic device 100 (e.g., the processor 220 of FIGS. 2 and 3) may extract geometric information of each of a plurality of vessels from at least one vascular image 410, 420, 430 (e.g., the vascular image 110 of FIG. 1). The geometric information may include at least one of a diameter, a length, a volume, a placement position, or a placement direction.

Before extracting the geometric information, the processor may classify a plurality of vessels 412, 422, 432 in the at least one vascular image 410, 420, 430. For example, the processor may classify the plurality of vessels in the at least one vascular image 410, 420, 430 by using a vessel-classification model. In an example, as illustrated in FIG. 4, the processor may classify three major coronary arteries-namely, a right coronary artery 432, a left anterior descending coronary artery 412, and a left circumflex coronary artery 422-in the at least one vascular image 410, 420, 430. In another example, the processor may broadly classify coronary arteries in the at least one vascular image 410, 420, 430 and may classify, in the at least one vascular image, a right coronary artery and a left main coronary artery.

Thereafter, the processor may extract geometric information of each of the plurality of vessels 412, 422, 432 from the at least one vascular image 410, 420, 430. For example, as illustrated in FIG. 4, the processor may obtain a diameter of a proximal portion 412a, 422a, 432a of each of the plurality of vessels 412, 422, 432 in the at least one vascular image 410, 420, 430.

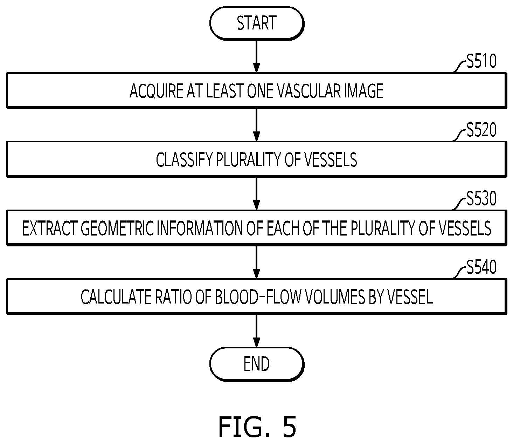

FIG. 5 is a diagram illustrating a method of calculating a ratio of blood-flow volumes by vessel using a vascular image. Referring to FIG. 5, a processor 220 (e.g., the processor 220 of FIGS. 2 and 3) of an electronic device 100 (e.g., the electronic device 100 of FIGS. 1 and 2) for calculating a ratio 120 of blood-flow volumes by vessel using a vascular image 110 (e.g., the vascular image 110 of FIG. 1) may, in step S510, acquire at least one vascular image. According to an example, the processor may acquire at least one vascular image captured from a target patient while a contrast agent is administered to the target patient. For example, the vascular image may include an image captured by coronary angiography.

In step S520, the processor may classify a plurality of vessels. For example, the processor may classify the plurality of vessels in at least one vascular image by using a vessel-classification model. In an example, the processor may classify a right coronary artery, a left anterior descending coronary artery, and a left circumflex coronary artery in at least one vascular image. In another example, the processor may classify a right coronary artery and a left main coronary artery in at least one vascular image.

In step S530, the processor may extract geometric information of each of the plurality of vessels. For example, the processor may extract geometric information of each of the plurality of vessels from at least one vascular image. The geometric information may include at least one of a diameter, a length, a volume, a placement position, or a placement direction.

In step S540, the processor may calculate a ratio of blood-flow volumes by vessel. For example, the processor may calculate the ratio of blood-flow volumes by vessel based on a value extracted as the geometric information of each of the plurality of vessels. According to an example, the processor may calculate a difference between a reference value for the geometric information of each of the plurality of vessels and a value extracted from at least one vascular image. Here, the reference value for the geometric information of each of the plurality of vessels may be set to an average of values calculated from a plurality of subjects. The processor may then calculate the ratio of blood-flow volumes by vessel for each of the plurality of vessels based on the calculated difference and reference distribution information of blood-flow volumes by vessel. Here, the reference distribution information of blood-flow volumes by vessel may be set to an average of values calculated from the plurality of subjects.

According to an example, the processor may estimate a blood-flow volume by vessel based on the ratio of blood-flow volumes by vessel. For example, the processor may set a weight according to the ratio of blood-flow volumes by vessel and may apply the weight to the blood-flow volume by vessel.

According to an example, the processor may provide a visual object corresponding to the ratio of blood-flow volumes by vessel. For example, the processor may provide, through a display, a visual object corresponding to the ratio of blood-flow volumes by vessel to a user. The visual object may include at least one of text, a symbol, an image, or an animation indicating the ratio of blood-flow volumes by vessel. For example, the processor may display the visual object corresponding to the ratio of blood-flow volumes by vessel so as to overlap a vascular image. In this case, the visual object corresponding to the ratio of blood-flow volumes by vessel may be displayed so as to overlap an area in which the corresponding vessel is placed or an adjacent area among the vessels included in the vascular image. In some implementations, when a vessel involved in calculation of the ratio of blood-flow volumes by vessel is not included in the vascular image, the processor may display the visual object corresponding to the ratio of blood-flow volumes by vessel for not only vessels included in the vascular image but also vessels not included.

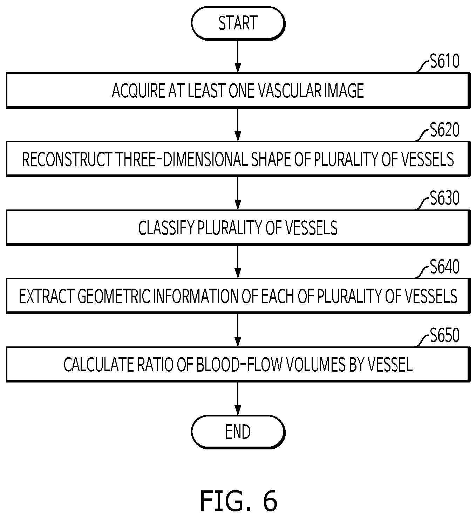

FIG. 6 is a diagram illustrating another method of calculating a ratio of blood-flow volumes by vessel using a vascular image. Referring to FIG. 6, a processor 220 (e.g., the processor 220 of FIGS. 2 and 3) of an electronic device 100 (e.g., the electronic device 100 of FIGS. 1 and 2) for calculating a ratio 120 of blood-flow volumes by vessel using a vascular image 110 (e.g., the vascular image 110 of FIG. 1) may, in step S610, acquire at least one vascular image. According to an example, the processor may acquire at least one vascular image captured from a target patient while a contrast agent is administered to the target patient. For example, the vascular image may include an image captured by coronary angiography.

In step S620, the processor may reconstruct a three-dimensional shape of a plurality of vessels. For example, the processor may reconstruct the three-dimensional shape of each of a plurality of vessels based on at least one vascular image.

In step S630, the processor may classify a plurality of vessels. For example, the processor may classify the plurality of vessels in each three-dimensional shape of the plurality of vessels by using a vessel-classification model.

In step S640, the processor may extract geometric information of each of the plurality of vessels. For example, the processor may extract geometric information of each of the plurality of vessels from each three-dimensional shape of the plurality of vessels. The geometric information may include at least one of a diameter, a length, a volume, an area, a placement position, or a placement direction.

In step S650, the processor may calculate a ratio of blood-flow volumes by vessel. For example, the processor may calculate the ratio of blood-flow volumes by vessel based on a value extracted as the geometric information of each of the plurality of vessels. According to an example, the processor may calculate a difference between a reference value for the geometric information of each of the plurality of vessels and a value extracted from at least one vascular image. Here, the reference value for the geometric information of each of the plurality of vessels may be set to an average of values calculated from a plurality of subjects. The processor may then calculate the ratio of blood-flow volumes by vessel for each of the plurality of vessels based on the calculated difference and reference distribution information of blood-flow volumes by vessel. Here, the reference distribution information of blood-flow volumes by vessel may be set to an average of values calculated from the plurality of subjects.

According to an example, the processor may estimate a blood-flow volume by vessel based on the ratio of blood-flow volumes by vessel. For example, the processor may set a weight according to the ratio of blood-flow volumes by vessel and may apply the weight to the blood-flow volume by vessel.

According to an example, the processor may provide a visual object corresponding to the ratio of blood-flow volumes by vessel. For example, the processor may provide, through a display, a visual object corresponding to the ratio of blood-flow volumes by vessel to a user. The visual object may include at least one of text, a symbol, an image, or an animation indicating the ratio of blood-flow volumes by vessel. For example, the processor may display the visual object corresponding to the ratio of blood-flow volumes by vessel so as to overlap a vascular image. In this case, the visual object corresponding to the ratio of blood-flow volumes by vessel may be displayed so as to overlap an area in which the corresponding vessel is placed or an adjacent area among the vessels included in the vascular image. In some implementations, when a vessel involved in calculation of the ratio of blood-flow volumes by vessel is not included in the vascular image, the processor may display the visual object corresponding to the ratio of blood-flow volumes by vessel for not only vessels included in the vascular image but also vessels not included.

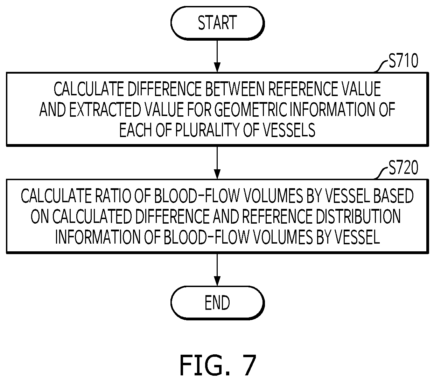

FIG. 7 is a diagram illustrating a method of calculating a ratio of blood-flow volumes by vessel using geometric information of each of a plurality of vessels. Referring to FIG. 7, a processor 220 (e.g., the processor 220 of FIGS. 2 and 3) of an electronic device 100 (e.g., the electronic device 100 of FIGS. 1 and 2) for calculating a ratio 120 of blood-flow volumes by vessel using a vascular image 110 (e.g., the vascular image 110 of FIG. 1) may, in step S710, calculate a difference between a reference value and an extracted value for the geometric information of each of a plurality of vessels. Here, the reference value for the geometric information of each of the plurality of vessels may be set to an average of values calculated from a plurality of subjects.

According to an example, the processor may calculate the difference as a ratio of the reference value for the geometric information of each of the plurality of vessels to the value extracted from at least one vascular image, as described in Equation 1 above. According to another example, the processor may calculate the difference as a ratio of a difference between the reference value for the geometric information of each of the plurality of vessels and the value extracted from at least one vascular image to the value extracted from at least one vascular image, as described in Equation 2 above.

In step S720, the processor may calculate a ratio of blood-flow volumes by vessel based on the calculated difference and reference distribution information of blood-flow volumes by vessel. For example, the processor may calculate the ratio of blood-flow volumes by vessel for each of the plurality of vessels based on the calculated difference between the reference value and the extracted value for the geometric information of each of the plurality of vessels and the reference distribution information of blood-flow volumes by vessel. Here, the reference distribution information of blood-flow volumes by vessel may be set to an average of values calculated from the plurality of subjects. For example, the processor may calculate a total blood-flow volume of a target patient based on the difference between the reference value and the extracted value for the geometric information of each of the plurality of vessels by using Equation 3. The processor may then calculate a blood-flow-volume ratio of each vessel by using the total blood-flow volume of the target patient through Equation 4.

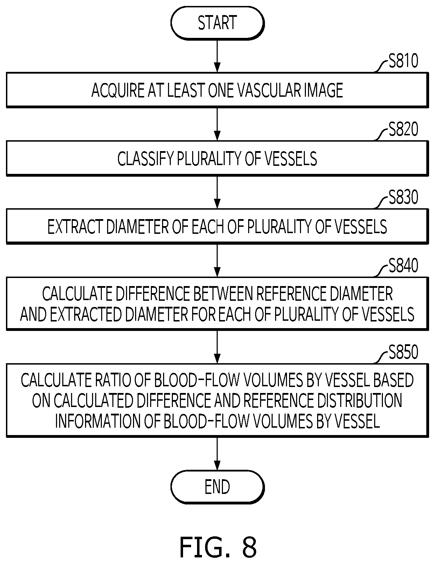

FIG. 8 is a diagram illustrating a method of calculating a ratio of blood-flow volumes by vessel using a diameter of each of a plurality of vessels. Referring to FIG. 8, a processor 220 (e.g., the processor 220 of FIGS. 2 and 3) of an electronic device 100 (e.g., the electronic device 100 of FIGS. 1 and 2) for calculating a ratio 120 of blood-flow volumes by vessel using a vascular image 110 (e.g., the vascular image 110 of FIG. 1) may, in step S810, acquire at least one vascular image. According to an example, the processor may acquire at least one vascular image captured from a target patient while a contrast agent is administered to the target patient. For example, the vascular image may include an image captured by coronary angiography.

In step S820, the processor may classify a plurality of vessels. For example, the processor may classify the plurality of vessels in at least one vascular image by using a machine-learning model (e.g., a vessel-classification model). In an example, the processor may classify a right coronary artery, a left anterior descending coronary artery, and a left circumflex coronary artery in at least one vascular image. In another example, the processor may classify a right coronary artery and a left main coronary artery in at least one vascular image.

In step S830, the processor may extract a diameter of each of the plurality of vessels. For example, the processor may measure a diameter of a proximal portion of each of the plurality of vessels in at least one vascular image.

In step S840, the processor may calculate a difference between a reference diameter and an extracted diameter for each of the plurality of vessels. Here, the reference diameter for each of the plurality of vessels may be set to an average of diameter values of each of the plurality of vessels calculated from a plurality of subjects. For example, a reference diameter of a left anterior descending coronary artery may be set to 4.0 mm, a reference diameter of a left circumflex coronary artery may be set to 3.94 mm, and a reference diameter of a right coronary artery may be set to 3.36 mm.

According to an example, the processor may calculate the difference between the reference value and the extracted value as a ratio of the reference diameter value of each of the plurality of vessels to the diameter value extracted from at least one vascular image by using Equation 1. According to another example, the processor may calculate the difference as a ratio of a difference between the reference diameter value of each of the plurality of vessels and the diameter value extracted from at least one vascular image to the diameter value extracted from at least one vascular image by using Equation 2.

In step S850, the processor may calculate a ratio of blood-flow volumes by vessel based on the calculated difference and reference distribution information of blood-flow volumes by vessel. For example, the processor may calculate the ratio of blood-flow volumes by vessel for each of the plurality of vessels based on the calculated difference between the reference diameter value and the extracted diameter value and the reference distribution information of blood-flow volumes by vessel. Here, the reference distribution information of blood-flow volumes by vessel may be set to an average of values calculated from the plurality of subjects.

According to an example, the processor may estimate a blood-flow volume by vessel based on the ratio of blood-flow volumes by vessel. For example, the processor may set a weight according to the ratio of blood-flow volumes by vessel and may apply the weight to the blood-flow volume by vessel.

According to an example, the processor may provide a visual object corresponding to the ratio of blood-flow volumes by vessel. For example, the processor may provide, through a display, a visual object corresponding to the ratio of blood-flow volumes by vessel to a user. The visual object may include at least one of text, a symbol, an image, or an animation indicating the ratio of blood-flow volumes by vessel. For example, the processor may display the visual object corresponding to the ratio of blood-flow volumes by vessel so as to overlap a vascular image. In this case, the visual object corresponding to the ratio of blood-flow volumes by vessel may be displayed so as to overlap an area in which the corresponding vessel is placed or an adjacent area among the vessels included in the vascular image. **In** some implementations, when a vessel involved in calculation of the ratio of blood-flow volumes by vessel is not included in the vascular image, the processor may display the visual object corresponding to the ratio of blood-flow volumes by vessel for not only vessels included in the vascular image but also vessels not included.

FIG. 9 illustrates an artificial-neural-network model according to an example of the present disclosure. Referring to FIG. 9, an artificial-neural-network model 900 is an example of a machine-learning model and may represent a statistical learning algorithm, or a structure executing the algorithm, that is implemented based on the structure of a biological neural network in machine-learning technology and cognitive science.

According to an example, the artificial-neural-network model 900 may represent a machine-learning model that acquires problem-solving capability by repeatedly adjusting synaptic weights of nodes, which are artificial neurons forming a network by synaptic connections like a biological neural network, so that an error between a correct output corresponding to a specific input and an inferred output is reduced. For example, the artificial-neural-network model 900 may include any probabilistic model or neural-network model used in artificial-intelligence learning methods such as machine learning, deep learning, etc.

According to an example, the above-described vessel-classification model and/or blood-flow-ratio-calculation model may be generated in the form of the artificial-neural-network model 900. For example, the artificial-neural-network model 900 may receive a vascular image captured from a vessel and may estimate a ratio of blood-flow volumes by vessel in the vascular image based on the received vascular image.

The artificial-neural-network model 900 may be implemented as a multi-layer perceptron composed of multiple layers of nodes and connections between the nodes. The artificial-neural-network model 900 according to the present example may be implemented by using one of neural-network structures that include a multi-layer perceptron. The artificial-neural-network model 900 may include an input layer 920 that receives input data 910 (or an input signal) from the outside, an output layer 940 that outputs output data 950 (or an output signal) corresponding to the input data 910, and n hidden layers 930_1 to 930_n (where n is a positive integer) located between the input layer 920 and the output layer 940 and extracting features from signals received from the input layer 920 and delivering the extracted features to the output layer 940. The output layer 940 may receive signals from the hidden layers 930_1 to 930_n and may output the signals to the outside.

Learning methods of the artificial-neural-network model 900 may include a supervised-learning method, in which the model is trained to be optimized for problem solving by inputting a teacher signal (i.e., a label) as a correct answer, and an unsupervised-learning method, in which the model does not require a teacher signal. According to an example, the electronic device 100 (e.g., the electronic device 100 of FIGS. 1 and 2) may train the artificial-neural-network model 900 by using a vascular image.

According to an example, the electronic device may generate training data to train the artificial-neural-network model 900. For example, the electronic device may generate a training-data set including vascular images. The electronic device may then train the artificial-neural-network model 900 to calculate a ratio of blood-flow volumes by vessel in a vascular image on the basis of the generated training-data set.

According to an example, an input variable of the artificial-neural-network model 900 may include a vascular image in which a vessel is captured. When the above-described input variable is input through the input layer 920, an output variable output from the output layer 940 of the artificial-neural-network model 900 may be a ratio of blood-flow volumes by vessel.

As such, a plurality of input variables and a plurality of output variables corresponding to the input variables are respectively matched with the input layer 920 and the output layer 940 of the artificial-neural-network model 900, and synaptic values between nodes included in the input layer 920, the hidden layers 930_1 to 930_n, and the output layer 940 are adjusted, so that a correct output corresponding to a specific input can be extracted. Through this learning process, hidden features in the input variable of the artificial-neural-network model 900 can be identified, and synaptic values (i.e., weights) between nodes of the artificial-neural-network model 900 can be adjusted such that an error between an output variable calculated on the basis of the input variable and a target output is reduced. The electronic device may train an algorithm that receives an image in which blood vessels are captured as an input by minimizing a loss between a calculated ratio of blood-flow volumes by vessel and annotation information for a ratio of blood-flow volumes by vessel. By using the artificial-neural-network model 900 trained in this manner, the ratio of blood-flow volumes by vessel can be estimated.

The flowchart and description above are merely examples and may be implemented differently in some examples. For example, in some examples, the order of respective steps may be changed, some steps may be repeatedly performed, some steps may be omitted, or some steps may be added.

The method described above may be provided as a computer program stored in a computer-readable recording medium for execution on a computer. The medium may be a type of medium that continuously stores a program executable by a computer, or temporarily stores the program for execution or download. In addition, the medium may be a variety of recording means or storage means having a single piece of hardware or a combination of several pieces of hardware, and is not limited to a medium that is directly connected to any computer system, and accordingly, may be present on a network in a distributed manner. An example of the medium includes a medium configured to store program instructions, including a magnetic medium such as a hard disk, a floppy disk, and a magnetic tape, an optical medium such as a CD-ROM and a DVD, a magnetic-optical medium such as a floptical disk, and a ROM, a RAM, a flash memory, etc. In addition, other examples of the medium may include an app store that distributes applications, a site that supplies or distributes various software, and a recording medium or a storage medium managed by a server.

The methods, operations, or techniques of the present disclosure may be implemented by various means. For example, these techniques may be implemented in hardware, firmware, software, or a combination thereof. Those skilled in the art will further appreciate that various illustrative logical blocks, modules, circuits, and algorithm steps described in connection with the disclosure herein may be implemented in electronic hardware, computer software, or combinations of both. To clearly illustrate this interchangeability of hardware and software, various illustrative components, blocks, modules, circuits, and steps have been described above generally in terms of their functionality. Whether such a function is implemented as hardware or software varies depending on design requirements imposed on the particular application and the overall system. Those skilled in the art may implement the described functions in varying ways for each particular application, but such implementation should not be interpreted as causing a departure from the scope of the present disclosure.

In a hardware implementation, processing units used to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computer, or a combination thereof.

Accordingly, various example logic blocks, modules, and circuits described in connection with the present disclosure may be implemented or performed with general purpose processors, DSPs, ASICs, FPGAs or other programmable logic devices, discrete gate or transistor logic, discrete hardware components, or any combination of those designed to perform the functions described herein. The general purpose processor may be a microprocessor, but in the alternative, the processor may be any related processor, controller, microcontroller, or state machine. The processor may also be implemented as a combination of computing devices, for example, a DSP and microprocessor, a plurality of microprocessors, one or more microprocessors associated with a DSP core, or any other combination of the configurations.

In the implementation using firmware and/or software, the techniques may be implemented with instructions stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, compact disc (CD), magnetic or optical data storage devices, etc. The instructions may be executable by one or more processors, and may cause the processor(s) to perform certain aspects of the functions described in the present disclosure.

When implemented in software, the techniques may be stored on a computer-readable medium as one or more instructions or codes, or may be transmitted through a computer-readable medium. The computer-readable media include both the computer storage media and the communication media including any medium that facilitates the transmission of a computer program from one place to another. The storage media may also be any available media that may be accessible to a computer. By way of non-limiting example, such a computer-readable medium may include RAM, ROM, EEPROM, CD-ROM or other optical disk storage, magnetic disk storage or other magnetic storage devices, or any other media that can be used to transmit or store desired program code in the form of instructions or data structures and can be accessible to a computer. In addition, any connection is properly referred to as a computer-readable medium.

For example, if the software is sent from a website, server, or other remote sources using coaxial cable, fiber optic cable, twisted pair, digital subscriber line (DSL), or wireless technologies such as infrared, wireless, and microwave, the coaxial cable, the fiber optic cable, the twisted pair, the digital subscriber line, or the wireless technologies such as infrared, wireless, and microwave are included within the definition of the medium. The disks and the discs used herein include CDs, laser disks, optical disks, digital versatile discs (DVDs), floppy disks, and Blu-ray disks, where disks usually magnetically reproduce data, while discs optically reproduce data using a laser. The combinations described above should also be included within the scope of the computer-readable media.

The software module may reside in RAM memory, flash memory, ROM memory, EPROM memory, EEPROM memory, registers, hard disk, removable disk, CD-ROM, or any other form of storage medium known. An exemplary storage medium may be connected to the processor such that the processor may read or write information from or to the storage medium. Alternatively, the storage medium may be integrated into the processor. The processor and the storage medium may exist in the ASIC. The ASIC may exist in the user terminal. Alternatively, the processor and storage medium may exist as separate components in the user terminal.

Although the examples described above have been described as utilizing aspects of the currently disclosed subject matter in one or more standalone computer systems, aspects are not limited thereto, and may be implemented in conjunction with any computing environment, such as a network or distributed computing environment. Furthermore, the aspects of the subject matter in the present disclosure may be implemented in multiple processing chips or apparatus, and storage may be similarly influenced across a plurality of apparatus. Such apparatus may include PCs, network servers, and portable apparatus.

Although the present disclosure has been described in connection with some examples herein, various modifications and changes can be made without departing from the scope of the present disclosure, which can be understood by those skilled in the art to which the present disclosure pertains. In addition, such modifications and changes should be considered within the scope of the claims appended herein.

**Claims**

1. A method performed by an apparatus comprising at least one processor (220), the method comprising:

   acquiring at least one vascular image (110);
   classifying, in the at least one vascular image (110), a plurality of vessels;
   extracting geometric information of each of the plurality of vessels from the at least one vascular image (110);
   identifying a ratio of blood-flow volumes by vessel (120) based on a value extracted as the geometric information of each of the plurality of vessels and a reference value for the geometric information of each of the plurality of vessels, wherein the reference value are set based on values calculated from a plurality of subjects; and
   outputting, in association with at least one of the acquired at least one vascular image (110), an indicator associated with the ratio of blood-flow volumes by vessel (120).

2. The method according to claim 1, wherein the geometric information comprises at least one of a diameter, a length, a volume, a placement position, or a placement direction.

3. The method according to claim 1, wherein the classifying of the plurality of vessels comprises:

   classifying, in the at least one vascular image (110), a right coronary artery, a left anterior descending coronary artery, and a left circumflex coronary artery; or
   classifying, in the at least one vascular image (110), the right coronary artery and a left main coronary artery.

4. The method according to claim 1, wherein the identifying of the ratio of blood-flow volumes by vessel (120) comprises:

   calculating a difference between the reference value and the extracted value for the geometric information of each of the plurality of vessels; and
   calculating, based on the calculated difference and reference distribution information of blood-flow volumes by vessel, the ratio of blood-flow volumes by vessel (120) for each of the plurality of vessels.

5. The method according to claim 4, wherein the difference comprises a ratio of the reference value to the extracted value or a ratio of a difference between the reference value and the extracted value to the extracted value.

6. The method according to claim 1, further comprising reconstructing, based on the at least one vascular image (110), a three-dimensional shape of the plurality of vessels.

7. The method according to claim 6, wherein the geometric information comprises at least one of a diameter, a length, a volume, an area, a placement position, or a placement direction.

8. The method according to claim 1, further comprising estimating, based on the ratio of blood-flow volumes by vessel (120), a blood-flow volume by vessel.

9. The method according to claim 1, further comprising providing a visual object corresponding to the identified ratio of blood-flow volumes by vessel (120).

10. The method according to claim 1, wherein the at least one vascular image (110) comprises an image captured by coronary angiography.

11. An electronic device (100) comprising:

   a memory (210) storing computer-readable instructions; and
   at least one processor (220) connected to the memory (210) and configured to execute the computer-readable instructions,
   wherein the computer-readable instructions, when executed by the at least one processor (220), are configured to cause the electronic device (100) to:

   acquire at least one vascular image (110);
   classify, in the at least one vascular image (110), a plurality of vessels;
   extract geometric information of each of the plurality of vessels from the at least one vascular image (110);

identify a ratio of blood-flow volumes by vessel (120) based on a value extracted as the geometric information of each of the plurality of vessels and a reference value for the geometric information of each of the plurality of vessels, wherein the reference value are set based on values calculated from a plurality of subjects; and output, in association with at least one of the acquired at least one vascular image (110), an indicator associated with the ratio of blood-flow volumes by vessel (120).

12. The electronic device (100) according to claim 11, wherein the computer-readable instructions, when executed by the at least one processor (220), are configured to cause the electronic device (100) to:

calculate a difference between the reference value and the extracted value for the geometric information of each of the plurality of vessels; and
calculate, based on the calculated difference and reference distribution information of blood-flow volumes by vessel, the ratio of blood-flow volumes by vessel (120) for each of the plurality of vessels.

13. The electronic device (100) according to claim 12, wherein the difference comprises a ratio of the reference value to the extracted value or a ratio of a difference between the reference value and the extracted value to the extracted value.

14. The electronic device (100) according to claim 11, wherein the computer-readable instructions, when executed by the at least one processor (220), are configured to cause the electronic device (100) to reconstruct, based on the at least one vascular image, a three-dimensional shape of the plurality of vessels.

15. A non-transitory computer-readable medium storing computer-readable instructions that, when executed by at least one processor (220), cause an electronic device (100) to:

acquire at least one vascular image (110);
classify, in the at least one vascular image (110), a plurality of vessels;
extract geometric information of each of the plurality of vessels from the at least one vascular image (110);
identify a ratio of blood-flow volumes by vessel (120) based on a value extracted as the geometric information of each of the plurality of vessels and a reference value for the geometric information of each of the plurality of vessels, wherein the reference value are set based on values calculated from a plurality of subjects; and
output, in association with at least one of the acquired at least one vascular image (110), an indicator associated with the ratio of blood-flow volumes by vessel (120).

| VASCULAR IMAGE<br>110 | → | ELECTRONIC DEVICE<br>100 | → | RATIO OF BLOOD-FLOW<br>VOLUMES BY VESSEL<br>120 |

FIG. 1

**ELECTRONIC DEVICE 100**

- MEMORY — 210
- INPUT-OUTPUT INTERFACE — 240
- PROCESSOR — 220
- COMMUNICATION MODULE — 230

FIG. 2

220

IMAGE ACQUISITION MODULE
310

VESSEL CLASSIFICATION MODULE
320

GEOMETRIC INFORMATION EXTRACTION MODULE
330

BLOOD FLOW RATIO CALCULATION MODULE
340

FIG. 3

FIG. 4

START

S510
ACQUIRE AT LEAST ONE VASCULAR IMAGE

S520
CLASSIFY PLURALITY OF VESSELS

S530
EXTRACT GEOMETRIC INFORMATION OF EACH OF THE PLURALITY OF VESSELS

S540
CALCULATE RATIO OF BLOOD-FLOW VOLUMES BY VESSEL

END

FIG. 5

START

S610
ACQUIRE AT LEAST ONE VASCULAR IMAGE

S620
RECONSTRUCT THREE-DIMENSIONAL SHAPE OF PLURALITY OF VESSELS

S630
CLASSIFY PLURALITY OF VESSELS

S640
EXTRACT GEOMETRIC INFORMATION OF EACH OF PLURALITY OF VESSELS

S650
CALCULATE RATIO OF BLOOD-FLOW VOLUMES BY VESSEL

END

# FIG. 6

START

S710

CALCULATE DIFFERENCE BETWEEN REFERENCE VALUE
AND EXTRACTED VALUE FOR GEOMETRIC INFORMATION OF
EACH OF PLURALITY OF VESSELS

S720

CALCULATE RATIO OF BLOOD-FLOW VOLUMES BY VESSEL BASED
ON CALCULATED DIFFERENCE AND REFERENCE DISTRIBUTION
INFORMATION OF BLOOD-FLOW VOLUMES BY VESSEL

END

FIG. 7

```
                    ┌─────────┐
                    │  START  │
                    └────┬────┘
                         │                              ╭─S810
        ┌────────────────▼───────────────────────────┐
        │     ACQUIRE AT LEAST ONE VASCULAR IMAGE     │
        └────────────────┬───────────────────────────┘
                         │                              ╭─S820
        ┌────────────────▼───────────────────────────┐
        │         CLASSIFY PLURALITY OF VESSELS       │
        └────────────────┬───────────────────────────┘
                         │                              ╭─S830
        ┌────────────────▼───────────────────────────┐
        │  EXTRACT DIAMETER OF EACH OF PLURALITY OF   │
        │                  VESSELS                    │
        └────────────────┬───────────────────────────┘
                         │                              ╭─S840
        ┌────────────────▼───────────────────────────┐
        │  CALCULATE DIFFERENCE BETWEEN REFERENCE     │
        │  DIAMETER AND EXTRACTED DIAMETER FOR EACH   │
        │          OF PLURALITY OF VESSELS            │
        └────────────────┬───────────────────────────┘
                         │                              ╭─S850
        ┌────────────────▼───────────────────────────┐
        │ CALCULATE RATIO OF BLOOD-FLOW VOLUMES BY    │
        │ VESSEL BASED ON CALCULATED DIFFERENCE AND   │
        │ REFERENCE DISTRIBUTION INFORMATION OF       │
        │ BLOOD-FLOW VOLUMES BY VESSEL                │
        └────────────────┬───────────────────────────┘
                         │
                    ┌────▼────┐
                    │   END   │
                    └─────────┘
```

## FIG. 8

FIG. 9

| | Europäisches Patentamt<br>European Patent Office<br>Office européen des brevets | **EUROPEAN SEARCH REPORT** | **Application Number**<br>EP 25 17 3257 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KR 2019 0075691 A (SAMSUNG LIFE PUBLIC WELFARE FOUNDATION [KR] ET AL.)<br>1 July 2019 (2019-07-01)<br>* paragraphs [0024], [0025], [0032], [0036], [0052] *<br>----- | 1-15 | INV.<br>A61B6/50<br>A61B6/00 |
| A | US 2014/200867 A1 (LAVI IFAT [IL] ET AL)<br>17 July 2014 (2014-07-17)<br>* the whole document *<br>----- | 1-15 | |
| A | US 2024/169540 A1 (BOUWMAN CHRIS [NL] ET AL) 23 May 2024 (2024-05-23)<br>* the whole document *<br>----- | 1-15 | |

| | TECHNICAL FIELDS SEARCHED (IPC) |
|---|---|
| | A61B |

The present search report has been drawn up for all claims

| Place of search | Date of completion of the search | Examiner |
|---|---|---|
| Munich | 18 September 2025 | Koprinarov, Ivaylo |

EPO FORM 1503 03.82 (P04C01)

## ANNEX TO THE EUROPEAN SEARCH REPORT
## ON EUROPEAN PATENT APPLICATION NO.

EP 25 17 3257

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2025

| Patent document cited in search report | Publication date | Patent family member(s) | | Publication date |
|---|---|---|---|---|
| KR 20190075691 A | 01-07-2019 | NONE | | |
| US 2014200867 A1 | 17-07-2014 | CN | 105190630 A | 23-12-2015 |
| | | EP | 2946319 A1 | 25-11-2015 |
| | | EP | 2946321 A1 | 25-11-2015 |
| | | EP | 3753494 A1 | 23-12-2020 |
| | | JP | 6542129 B2 | 10-07-2019 |
| | | JP | 6636331 B2 | 29-01-2020 |
| | | JP | 6790179 B2 | 25-11-2020 |
| | | JP | 7039442 B2 | 22-03-2022 |
| | | JP | 2016509501 A | 31-03-2016 |
| | | JP | 2016511649 A | 21-04-2016 |
| | | JP | 2019088792 A | 13-06-2019 |
| | | JP | 2019193808 A | 07-11-2019 |
| | | JP | 2021045558 A | 25-03-2021 |
| | | JP | 2022169579 A | 09-11-2022 |
| | | JP | 2024153627 A | 29-10-2024 |
| | | KR | 20150110609 A | 02-10-2015 |
| | | US | 2014200867 A1 | 17-07-2014 |
| | | US | 2017364658 A1 | 21-12-2017 |
| | | US | 2018268941 A1 | 20-09-2018 |
| | | US | 2019164649 A1 | 30-05-2019 |
| | | US | 2019385745 A1 | 19-12-2019 |
| | | WO | 2014111927 A1 | 24-07-2014 |
| | | WO | 2014111929 A1 | 24-07-2014 |
| | | WO | 2014111930 A1 | 24-07-2014 |
| US 2024169540 A1 | 23-05-2024 | EP | 4622554 A1 | 01-10-2025 |
| | | US | 2024169540 A1 | 23-05-2024 |
| | | WO | 2024110438 A1 | 30-05-2024 |

EPO FORM P0459